(19) Europäisches Patentamt
European Patent Office
Office européen des brevets

(11) EP 1 455 737 B1

(12) EUROPÄISCHE PATENTSCHRIFT

(45) Veröffentlichungstag und Bekanntmachung des Hinweises auf die Patenterteilung:
**07.06.2006 Patentblatt 2006/23**

(21) Anmeldenummer: **02805307.2**

(22) Anmeldetag: **06.12.2002**

(51) Int Cl.:
***A61Q 5/06*** *(2006.01)* ***A61Q 19/00*** *(2006.01)*

(86) Internationale Anmeldenummer:
**PCT/EP2002/013852**

(87) Internationale Veröffentlichungsnummer:
**WO 2003/053398 (03.07.2003 Gazette 2003/27)**

(54) **OBERFLÄCHENMODIFZIERTES ZINKOXID ZUR HERSTELLUNG NANOPARTIKULÄRER DISPERSIONEN**

SURFACE MODIFIED ZINC OXIDE FOR THE PRODUCTION OF NANOPARTICULATE DISPERSIONS

OXYDE DE ZINC A SURFACE MODIFIEE, SERVANT A LA PRODUCTION DE DISPERSIONS NANOPARTICULAIRES

(84) Benannte Vertragsstaaten:
**AT BE BG CH CY CZ DE DK EE ES FI FR GB GR IE IT LI LU MC NL PT SE SI SK TR**

(30) Priorität: **21.12.2001 DE 10163256**

(43) Veröffentlichungstag der Anmeldung:
**15.09.2004 Patentblatt 2004/38**

(73) Patentinhaber:
- **Henkel Kommanditgesellschaft auf Aktien 40589 Düsseldorf (DE)**
- **Sus Tech GmbH & Co. KG 64287 Darmstadt (DE)**

(72) Erfinder:
- **KLISS, Rainer 64354 Reinheim (DE)**
- **ELSÄSSER, Ralf 86153 Augsburg (DE)**
- **HELLER, Melita 40591 Düsseldorf (DE)**
- **UMBREIT, Christian 40589 Düsseldorf (DE)**
- **HAHN, Horst 64342 Seeheim-Jungenheim (DE)**
- **KROPF, Christian 40721 Hilden (DE)**
- **BERBER, Mete 64372 Ober-Ramstadt (DE)**
- **BULTO CARULLA, Victor 08034 Barcelona (ES)**

(56) Entgegenhaltungen:
**EP-A- 1 112 964** **WO-A-00/50503**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein oberflächenmodifiziertes nanoskaliges Zinkoxid, wobei die Oberflächenmodifikation eine Beschichtung mit einer Oligo- oder Polyethylenglycolsäure umfaßt. Dieses oberflächenmodifizierte Zinkoxid zeichnet sich dadurch aus, daß es in einem flüssigen Medium stabile Dispersionen bildet. Weiterhin betrifft die vorliegende Erfindung ein Verfahren zur Herstellung von oberflächenmodifiziertem Zinkoxid sowie ein Verfahren zur Herstellung von nanoskaligen Zinkoxiddispersionen. Derartige Zinkoxide oder Zinkoxiddispersionen können unterschiedliche technische Verwendung finden wie z.B. den Einsatz in kosmetischen Rezepturen, als UV-Schutz oder als antimikrobieller Wirkstoff.

Bekannt ist die Herstellung von Zinkoxid durch trockene und nasse Verfahren. Die klassische Methode der Verbrennung von Zink, die als trockenes Verfahren bekannt ist (z.B. Gmelin Band 32, 8. Aufl., Ergänzungsband, S. 772 ff.), erzeugt aggregierte Partikel mit einer breiten Größenverteilung. Zwar ist es grundsätzlich möglich, durch Mahlverfahren Teilchengrößen im Submikrometerbereich herzustellen, doch aufgrund der zu geringen erzielbaren Scherkräfte sind aus solchen Pulvern Dispersionen mit mittleren Teilchengrößen im unteren Nanometerbereich nicht erzielbar. Besonders feinteiliges Zinkoxid wird vor allem naßchemisch durch Fällungsprozesse hergestellt. Die Fällung in wässriger Lösung liefert in der Regel hydroxid- und/oder carbonathaltige Materialien, die thermisch zu Zinkoxid umgesetzt werden müssen. Die thermische Nachbehandlung wirkt sich dabei auf die Feinteiligkeit negativ aus, da die Partikel dabei Sinterprozessen unterworfen sind, die zur Bildung mikrometergroßer Aggregate führen, die durch Mahlung nur unvollständig auf die Primärpartikel heruntergebrochen werden können.

Nanopartikuläre Metalloxide können beispielsweise durch das Mikroemulsionsverfahren erhalten werden. Bei diesem Verfahren wird eine Lösung eines Metallalkoxids zu einer Wasser-in-Öl-Mikroemulsion getropft. In den inversen Micellen der Mikroemulsion, deren Größe im Nanometerbereich liegt, findet dann die Hydrolyse der Alkoxide zum nanopartikulären Metalloxid statt. Die Nachteile dieses Verfahrens liegen insbesondere darin, daß die Metallalkoxide teure Ausgangsstoffe darstellen, daß zusätzlich Emulgatoren verwendet werden müssen und daß die Herstellung der Emulsionen mit Tröpfchengrößen im Nanometerbereich einen aufwendigen Verfahrensschritt darstellt.

In der DE 199 07 704 wird ein nanoskaliges über eine Fällungsreaktion hergestelltes Zinkoxid beschrieben. Hierbei wird das nanoskalige Zinkoxid ausgehend von einer Zinkacetatlösung über eine alkalische Fällung hergestellt. Das abzentrifugierte Zinkoxid kann durch Zugabe von Methylenchlorid zu einem Sol redispergiert werden. Die so hergestellten Zinkoxiddispersionen haben den Nachteil, daß sie aufgrund fehlender Oberflächenmodifizierung keine gute Langzeitstabilität besitzen. Ebenfalls aufgrund der fehlenden Oberflächenmodifizierung beschränkt sich die Herstellung von Zinkoxiddispersionen auf nicht mit Wasser mischbare Dispergierungsmittel. In der Beschreibung wird weiterhin noch die Möglichkeit zur Herstellung von kolloiddispersem Fällungszinkoxid erwähnt, bei dem ein Diol-Polyol-WasserGemisch unter Verwendung von Oberflächenmodifikatoren, wie beispielsweise Triethanolamin, eingesetzt wird. In der WO 00/50503, die die Priorität dieser deutschen Anmeldung in Anspruch nimmt, wird ein Beispiel hierzu formuliert, wobei eine Mischung dieser Komponenten in einem Verhältnis Ethylenglycol : Wasser : Triethanolamin im Gewichtsverhältnis 2 : 1 : 0,55 eingesetzt wird. Diese Mischung weist aufgrund des hohen Anteils an nicht wässrigen Komponenten deutliche Nachteile gegenüber den reinen wässrigen Dispersionen auf. Darüber hinaus werden die Zinkoxidpartikel hierbei nicht mit einer permanenten Oberflächenbeschichtung ausgestattet.

In der WO 00/50503 werden Zinkoxidgele, die nanoskalige Zinkoxidpartikel mit einem Partikeldurchmesser von $\leq$15 nm enthalten und die Solen redispergierbar sind, beschrieben. Hierbei werden die durch basische Hydrolyse einer Zinkverbindung in Alkohol oder in einem Alkohol/Wassergemisch hergestellten Fällungen durch Zugabe von Dichlormethan oder Chloroform redispergiert. Nachteilig ist hierbei, dass in Wasser oder in wäßrigen Dispergierungsmitteln keine stabilen Dispersionen erhalten werden.

In der Veröffentlichung aus Chem. Mater "Synthesis and Characterization of Poly(vinylpyrrolidone)-Modified Zinc Oxide Nanoparticles, Lin Guo and Shihe Yang, 2000, <u>12</u>" werden Wurtzit Zinkoxidnanopartikel mit Polyvinylpyrrolidon oberflächenbeschichtet. Der Nachteil hierbei ist, daß mit Polyvinylpyrrolidon beschichtete Zinkoxidpartikel nicht in Wasser dispergierbar sind.

In der WO 93/21127 wird ein Verfahren zur Herstellung oberflächenmodifizierter nanoskaliger keramischer Pulver beschrieben. Hierbei wird ein nanoskaliges keramisches Pulver durch Aufbringen einer niedrigmolekülaren organischen Verbindung, beispielsweise Propionsäure, oberflächenmodifiziert. Dieses Verfahren kann nicht zur Oberflächenmodifizierung von Zinkoxid eingesetzt werden, da die Modifizierungsreaktionen in wäßriger Lösung durchgeführt werden und Zinkoxid sich in wäßrigen organischen Säuren auflöst. Daher läßt sich dieses Verfahren nicht zur Herstellung von Zinkoxiddispersionen anwenden; darüber hinaus ist Zinkoxid in dieser Anmeldung auch nicht als mögliches Ausgangsmaterial für nanoskalige keramische Pulver genannt.

Der vorliegenden Erfindung lag daher die Aufgabe zugrunde, ein nanoskaliges Zinkoxid bereitzustellen, das die Herstellung stabiler nanopartikulärer Dispersionen in Wasser oder polaren organischen Lösemitteln erlaubt. Um einen Einsatz in beispielsweise kosmetischen Rezepturen in technischem Maßstab realisieren zu können, ist es hierzu notwendig, von kommerziell verfügbaren, kostengünstigen Edukten für die Herstellung auszugehen, wobei das Herstellungsverfahren im weiteren eine leichte Abtrennung von Nebenprodukten ermöglichen soll. Eine irreversible Aggregation der Partikel soll nach Möglichkeit vermieden werden, damit ein aufwendiger Mahlprozeß vermieden werden kann.

Der Erfindung liegt die Erkenntnis zugrunde, daß durch eine Oberflächenmodifikation von Zinkoxid mit einer Oligo- oder Polyethylenglycolsäure, eine Langzeitstabilität von Dispersionen des oberflächenmodifizierten Zinkoxids erreicht werden kann.

Gegenstand der vorliegenden Erfindung ist daher ein oberflächenmodifiziertes nanopartikuläres Zinkoxid, das dadurch gekennzeichnet ist, daß die Oberflächenmodifikation eine Beschichtung mit einer Oligo- oder Polyethylenglycolsäure umfaßt.

Überraschenderweise bildet dieses oberflächenmodifizierte nanopartikuläre Zinkoxid in einem flüssigen Medium langzeitstabile Dispersionen. Für die Herstellung des Oberflächenmodifizierten Zinkoxids kann frei erhältliches Zinkoxidpulver eingesetzt werden, wobei die primäre Kristallitgröße im nanopartikulären Bereich liegen muß. Hierunter sind Teilchen zu verstehen, die einen volumengewichteten mittleren Kristallitdurchmesser von weniger als 1000 nm aufweisen, insbesondere Teilchen, die einen Durchmesser von weniger als 500 nm aufweisen. Die volumengewichtete mittlere Kristallitgröße ist mit Röntgenbeugungsverfahren, insbesondere über eine Scherrer-Analyse bestimmbar. Das Verfahren ist beispielsweise beschrieben in: C. E. Krill, R. Birringer: "Measuring average grain sizes in nanocrystalline materials", Phil. Mag. A 77, S. 621 (1998). Demnach kann die volumengewichtete mittlere Kristallitgröße D bestimmt werden durch den Zusammenhang

$$D = K\lambda/\beta\cos\theta.$$

Dabei ist $\lambda$ die Wellenlänge der verwendeten Röntgenstrahlung, $\beta$ ist die volle Breite auf halber Höhe des Reflexes an der Beugungsposition $2\theta$. K ist eine Konstante der Größenordnung 1, deren genauer Wert von der Kristallform abhängt. Man kann diese Unbestimmtheit von K vermeiden, indem man die Linienverbreiterung als integrale Weite $\beta_i$ bestimmt, wobei $\beta_i$ definiert ist als die Fläche unter dem Röntgenbeugungsreflex, geteilt durch dessen maximaler Intensität $I_0$:

$$\beta_i = \frac{1}{I_0}\int_{2\theta_1}^{2\theta_2} I(2\theta)d(2\theta)$$

Dabei sind die Größen $2\theta_1$ und $2\theta_2$ die minimale und maximale Winkelposition des Bragg-Reflexes auf der $2\theta$-Achse. $I(2\theta)$ ist die gemessene Intensität des Reflexes als Funktion von $2\theta$. Unter Verwendung von diesem Zusammenhang ergibt sich als Gleichung zur Bestimmung der volumengewichteten mittleren Kristallitgröße D:

$$D = \lambda/\beta_i\cos\theta$$

Dieses Zinkoxid kann direkt mit einer Oligo- oder Polyethylenglycolsäure oberflächenmodifiziert werden, oder zunächst einem Aktivierungsschritt unterzogen werden. Die Oberflächenaktivierung des Zinkoxids kann beispielsweise durch Versetzen mit einer stark verdünnten Säure oder Base erfolgen. Besonders gut geeignet ist die Verwendung von amorphen oder kristallinen Zinkoxiden, die über ein elektrochemisches Verfahren, das in der WO 00/14302 beschrieben ist, erhalten wird. Bei diesem Verfahren werden Metalle anodisch aufgelöst und an der Katodenseite als Metalloxide ausgefällt. Ermöglicht wird dies durch die Verwendung organischer Elektrolyte mit einem geringen Wassergehalt unter gleichzeitigem Zusatz von Leitsalzen. Beim Einsatz dieser Zinkoxide hat es sich als besonders vorteilhaft herausgestellt, wenn die Zinkoxide vor der Oberflächenmodifizierung nicht getrocknet werden, sondern als Zinkoxid-Suspension eingesetzt werden.

Ein weiterer Gegenstand der Erfindung ist ein Verfahren zur Herstellung von oberflächenmodifiziertem nanopartikulären Zinkoxid, das dadurch gekennzeichnet ist, daß unbehandeltes Zinkoxid in einem polaren Lösemittel suspendiert wird, danach mit einer Oligo- oder Polyethylenglycolsäure versetzt und erhitzt wird und

das polare Lösemittel entfernt wird. In diesen polaren organischen Lösemitteln kann auch Wasser in einem beliebigen Mischungsverhältnis enthalten sein.

Ein weiterer Gegenstand der Erfindung ist ein Verfahren zur Herstellung von Zinkoxiddispersionen, das dadurch gekennzeichnet ist, daß oberflächenmodifiziertes Zinkoxid in Wasser, in ein organisches Lösemittel oder in eine Mischung

aus einem organischen Lösemittel und Wasser eingebracht und durch ein geeignetes Verfahren dispergiert wird.

Gegenstand der Erfindung sind weiterhin Zinkoxiddispersionen, die nach dem oben angegebenen erfindungsgemäßen Verfahren hergestellt sind und die dadurch gekennzeichnet sind, daß die Dispersionen einen Gehalt an dispergiertem Zinkoxid von 0,001 bis 50 % aufweisen.

Ein weiterer Gegenstand der vorliegenden Erfindung ist ein kosmetisches Mittel, das ein erfindungsgemäß oberflächenbeschichtetes Zinkoxid oder eine Zinkoxiddispersion enthält.

Ein weiterer Gegenstand der vorliegenden Erfindung ist die Verwendung von oberflächenmodifiziertem Zinkoxid oder Zinkoxiddispersionen, die nach dem erfindungsgemäßen Verfahren hergestellt sind:

- zum UV-Schutz
- als antimikrobieller Wirkstoff

Gemäß einer bevorzugten Ausführungsform der vorliegenden Erfindung ist das oberflächenmodifizierte Zinkoxid in einem flüssigen Medium redispergierbar und bildet stabile Dispersionen. Dies ist besonders vorteilhaft, weil die aus dem erfindungsgemäßen Zinkoxid hergestellten Dispersionen vor der Weiterverarbeitung nicht erneut dispergiert werden müssen, sondern direkt verarbeitet werden können.

Nach einer bevorzugten Ausführungsform der vorliegenden Erfindung ist das oberflächenmodifizierte Zinkoxid in polaren organischen Lösemitteln redispergierbar und bildet stabile Dispersionen. Dies ist besonders vorteilhaft, da hierdurch eine gleichmäßige Einarbeitung beispielsweise in Kunststoffe oder Folien möglich ist.

Nach einer weiteren bevorzugten Ausführungsform der vorliegenden Erfindung ist das oberflächenmodifizierte Zinkoxid in Wasser redispergierbar und bildet dort stabile Dispersionen. Dies ist besonders vorteilhaft, da sich hierdurch die Möglichkeit eröffnet, das erfindungsgemäße Material beispielsweise in kosmetischen Rezepturen einzusetzen, wobei der Verzicht auf organische Lösemittel einen großen Vorteil darstellt. Denkbar sind auch Mischungen von Wasser und polaren organischen Lösemitteln.

In einer weiteren bevorzugten Ausführungsform der vorliegenden Erfindung entspricht die zur Oberflächenmodifizierung des Zinkoxids Oligo- oder Polyethylenglycolsäure der allgemeinen Form $R-CH_2-(O-CH_2-CH_2)_n-O-CH_2-COOH$, wobei n eine ganze Zahl von 0 bis 40 ist. Dies ist besonders vorteilhaft, da durch den Einsatz einer Verbindung dieses Typs einerseits eine Redispergierbarkeit in polaren organischen Lösemitteln sowie in Wasser ermöglicht wird, jedoch andererseits nicht wie beim Einsatz von ansonsten für diesen Zweck eingesetzten Fettsäuren die nanopartikulären Zinkoxide aufgelöst werden.

Nach einer weiteren besonders bevorzugten Ausführungsform der vorliegenden Erfindung ist der Rest R ausgewählt aus H, $CH_3$, $C_2H_5$, $C_3H_7$, $CH(CH_3)_2$, OH, $NH_2$, COOH, $CONH_2$, $CO_2CH_3$, $CO_2C_2H_5$, $CO_2C_3H_7$ und $CO_2$ CH $(CH_3)_2$. Dies ist besonders vorteilhaft, da bei der Wahl dieser Substituenten einerseits das Oberflächenmodifikationsmittel gut auf der Oberfläche des nanopartikulären Zinkoxids haftet und andererseits durch die sterischen und elektrostatischen Eigenschaften besonders gleichmäßige Oberflächenmodifikationen liefern.

Nach einer weiteren bevorzugten Ausführungsform der vorliegenden Erfindung umfaßt die Oberflächenmodifizierung des Zinkoxids eine Oligo- bzw. Polyethylenglycol-Di-Essigsäure der allgemeinen Formel $HOOC-CH_2-(O-CH_2-CH_2)_n-O-CH_2-COOH$, wobei n eine ganze Zahl von 0 bis 40 ist. Der Einsatz von Verbindungen dieses Typs ist besonders vorteilhaft, da hierdurch einerseits eine feste Bindung an das nanopartikuläre Zinkoxid erreicht wird und andererseits eine gute Redispergierbarkeit in polaren organischen Lösemitteln sowie in Wasser gewährleistet ist. Ein weiterer Vorteil dieser Verbindungskategorie ist, daß viele Vertreter der Oligo- bzw. Polyethylenglycol-Di-Essigsäure in kosmetischen Rezepturen eingesetzt werden können, da sie keine Toxizität aufweisen und bereits zugelassen sind.

Nach einer weiteren besonders bevorzugten Ausführungsform der vorliegenden Erfindung wird die Oberfläche der Zinkoxidpartikel mit Polyethylenglycoldisäure600 modifiziert. Dies ist besonders vorteilhaft, weil Polyethylenglycoldisäure600 (mit n=11 in der allgemeinen Formel $HOOC-CH_2-(O-CH_2-CH_2)_n-O-CH_2-COOH$) für kosmetische Zwecke bereits zugelassen ist und außerdem von diesem Mittel bereits eine verhältnismäßig Geringe Menge ausreicht, um eine gute Redispergierbarkeit des modifizierten Zinkoxids zu erreichen.

Nach einer besonders bevorzugten Ausführungsform der vorliegenden Erfindung wird die Oberfläche der Zinkoxidpartikel mit 2-[2-(2-Methoxyethoxy)-ethoxy]-essigsäure modifiziert. Der Einsatz dieser Substanz zur Oberflächenmodizierung von nanopartikulärem Zinkoxid ist daher besonders vorteilhaft, weil diese Substanz einerseits sehr preisgünstig ist und des weiteren eine sehr gute Oberflächenadhäsion aufweist.

Gemäß einer bevorzugten Ausführungsform der vorliegenden Erfindung besitzen die oberflächenmodifizierten Zinkoxidpartikel einen Durchmesser von 1 bis 200 nm. Dies ist besonders vorteilhaft, da innerhalb dieser Größenverteilung eine gute Redispergierbarkeit gewährleistet ist.

Gemäß einer besonders bevorzugten Ausführungsform der vorliegenden Erfindung weisen die Zinkoxidnanopartikel einen Durchmesser von 2 bis 50 nm, ganz besonders bevorzugt 3 bis 10 nm auf. Dieser Größenbereich ist besonders vorteilhaft, da nach Redispergierung von solchen Zinkoxidnanopartikeln die entstehenden Dispersionen

transparent sind und somit beispielsweise bei Zugabe zu kosmetischen Rezepturen die Farbgebung nicht beeinflussen. Darüber hinaus ergibt sich hierdurch auch die Möglichkeit zum Einsatz in transparenten Folien. Wenn die Zinkoxiddispersionen als UV-Absorber eingesetzt werden sollen, ist es ratsam, Partikel mit einem Durchmesser von mehr als 5 nm einzusetzen, da unterhalb dieser Grenze eine Verschiebung der Absorptionskante in den kurzwelligen Bereich erfolgt (L. Brus, J. Phys., Chem. (1986), 90, 2555 - 2560).

Ein weiterer Gegenstand der vorliegenden Erfindung ist ein Verfahren zur Herstellung von oberflächenmodifiziertem Zinkoxid, das dadurch gekennzeichnet ist, daß man das unbehandelte Zinkoxid in einem polaren Lösemittel suspendiert, danach mit einer Oligo- oder Polyethylenglycolsäure zur Oberflächenmodifizierung versetzt und erhitzt und anschließend das polare Lösemittel entfernt. Unter einem polaren Lösemittel sind hierbei sowohl polare organische Lösemittel, sowie Wasser und ebenfalls Mischungen aus organischem polaren Lösemittel und Wasser in beliebigen Verhältnissen zu verstehen. Zur Oberflächenmodifizierung geeignete Oligo- oder Polyethylenglycolsäuren besitzen die allgemeine Form $R-CH_2-(O-CH_2-CH_2)_n-O-CH_2-COOH$, wobei n eine ganze Zahl von 0 bis 40 ist und R ausgewählt ist aus H, $CH_3$, $C_2H_5$, $C_3H_7$, $CH(CH_3)_2$, OH, $NH_2$, COOH, $CONH_2$, $CO_2CH_3$, $CO_2C_2H_5$, $CO_2C_3H_7$ und $CO_2$ $CH(CH_3)_2$. Besonders gut geeignet sind hierbei Oligo- bzw. Polyethylenglycol-Di-Essigsäuren der allgemeinen Formel $HOOC-CH_2-(O-CH_2-CH_2)_n-O-CH_2-COOH$, wobei n eine ganze Zahl von 0 bis 40 ist. Ein ebenfalls bevorzugtes Oberflächenmodifizierungsmittel zur Durchführung des erfindungsgemäßen Verfahrens ist die 2-[2-(2-Methoxyethoxy)-ethoxy]-essigsäure. Der Vorteil dieses erfindungsgemäßen Verfahrens ist es, daß als Produkt oberflächenmodifizierte Zinkoxide erhalten werden, die in einem flüssigen Medium sehr gut redispergierbar sind und stabile Dispersionen liefern.

Nach einer besonders bevorzugten Ausführungsform des erfindungsgemäßen Verfahrens wird das Lösemittel durch Verdampfen unter Normaldruck oder Unterdruck, durch Ausfrieren, Gefriertrocknen, Abfiltrieren und anschließendem Trocknen oder Trocknen bei erhöhter Temperatur bei Normaldruck oder bevorzugt bei vermindertem Druck entfernt. Dies ist besonders vorteilhaft, da hierdurch das Verfahren einerseits beschleunigt wird und andererseits ein schonender Umgang mit dem oberflächenmodifizierten, nanopartikulären Zinkoxid gewährleistet ist, sowie eine Möglichkeit der Lösemittelrückgewinnung gegeben wird.

Ein weiterer Gegenstand der vorliegenden Erfindung ist ein Verfahren zur Herstellung von Zinkoxiddispersionen, das dadurch gekennzeichnet ist, daß man ein erfindungsgemäß oberflächenmodifiziertes Zinkoxid in Wasser, in ein organisches Lösemittel oder in eine Mischung aus einem organischen Lösemittel und Wasser einbringt und durch ein geeignetes Verfahren dispergiert. Hierzu geeignete Verfahren können sein Rühren, Schütteln, Ultraschallbehandlung, Erwärmen und/oder Einsatz kommerzieller Dispergierapparate wie Ultra-Turrax, Dissolver, Perlmühle. Dies ist besonders vorteilhaft, da bei diesem Verfahren auch getrocknete, erfindungsgemäß oberflächenmodifiziertes Zinkoxidpulver eingesetzt werden können und in den angegebenen Lösemitteln bzw. Lösemittelgemischen nahezu vollständig wieder redispergiert werden. Hierdurch können bei Verwendung der gleichen Ausgangssubstanz durch Wahl des Dispergierungsmittels verschiedenartige Zinkoxiddispersionen hergestellt werden, die auf die unterschiedlichen Anwendungsgebiete abgestimmt sind.

Nach einer bevorzugten Ausführungsfom des erfindungsgemäßen Verfahrens zur Herstellung von Zinkoxiddispersionen wird ein organisches Lösemittel mit einem Dipolmoment $\geq 0{,}35\ \mu/D$ eingesetzt. Dies ist besonders vorteilhaft, da durch die Wahl eines Dispergierungsmittels mit einem solchen Dipolmoment einerseits die Langzeitstabilität der Dispersionen gewährleistet ist und andererseits hierdurch Zinkoxiddispersionen hergestellt werden, die sich beispielsweise zum Einsatz in Kunststoffen eignen.

Nach einer weiteren bevorzugten Ausführungsform des erfindungsgemäßen Verfahrens zur Herstellung Zinkoxiddispersionen ist das organische Lösemittel ausgewählt oder eine Mischung aus Methanol, n-Propanol, i-Propanol, Aceton, Diethylether, Dimethylsulfoxid, Tetrahydrofuran, Methylenchlorid, Trichlormethan, Ethanol, Ethylacetat, Isobutylacetat und/oder Toluol. Dies ist besonders vorteilhaft, da durch dieses Verfahren Zinkoxiddispersionen in Flüssigkeiten unterschiedlicher physikalischer Eigenschaften hergestellt werden können, wodurch sich ein breites Anwendungsspektrum für weitere Verwendungen dieser Zinkoxiddispersionen ergibt. Als relevante physikalische Eigenschaften des Lösemittels kommen die Wassermischbarkeit, Protizität/Aprotizität, Dipolmoment, Siedetemperatur oder Schmelzpunkt in Frage.

Ein weiterer Gegenstand der vorliegenden Erfindung sind Zinkoxiddispersionen, die nach einem oben angegebenen Verfahren hergestellt wurden und die dadurch gekennzeichnet sind, daß die Dispersionen einen Gehalt an dispergiertem Zinkoxid von 0,001 bis 50 Gew.-% aufweisen. Dies ist besonders vorteilhaft, da hierdurch Zinkoxiddispersionen mit einem breiten Konzentrationsbereich bereitgestellt werden und somit für diverse Einsatzzwecke geeignet sind.

Gemäß einer bevorzugten Ausführungsform der erfindungsgemäßen Zinkoxiddispersionen haben die Dispersionen einen Gehalt an dispergiertem Zinkoxid von 0,1 bis 10 Gew.-%, besonders bevorzugt 1 bis 5 Gew.-%. Dispersionen mit einem solchen Gehalt an Zinkoxid haben den besonderen Vorteil, daß sie selbst bei widrigen äußeren Umständen stabil bleiben und es nicht zu einem Ausfallen des dispergierten Oxides kommt. Widrige Umstände sind in diesem Zusammenhang Umgebungstemperaturen in einem Bereich von mehr als +/- 10 °C Abweichung von der Raumtemperatur oder mechanische Belastungen wie Vibrationen oder Rühren.

Eine besonders bevorzugte Ausführungsform der erfindungsgemäßen Zinkoxiddispersionen zeichnet sich dadurch aus, daß die Dispersionen weitestgehend transparent sind. Dies ist besonders vorteilhaft, da hierdurch die Zinkoxiddispersionen bei der Einarbeitung in andere Produkte wie z.B. Kunststoffformteile keinen Einfluß auf die Farbgebung nehmen. Insbesondere können die Zinkoxiddispersionen in Folien eingesetzt werden, da sie die Transparanz dieser Folien ebenfalls nicht beeinträchtigen.

Ein weiterer Gegenstand der vorliegenden Erfindung ist ein Verfahren zur Oberflächenbeschichtung mit dem erfindungsgemäß oberflächenmodifizierten Zinkoxid oder einer Dispersion hieraus das dadurch gekennzeichnet ist, daß man eine Dispersion der erfindungsgemäß oberflächenmodifizierten Zinkoxidpartikel auf die zu beschichtende Oberfläche aufträgt und das Dispergierungsmittel anschließend entfernt. Die Entfernung des Dispergierungsmittels kann auf verschiedene Arten vollzogen werden: Erwärmen, Abziehen des Dispergierungsmittels unter Vakuum evtl. bei gleichzeitigem Erwärmen, Gefriertrocknung, Lufttrocknung, Heißlufttrocknung, UV- und Infrarottrocknung oder Hochfrequenztrocknung. Das Beschichtungsverfahren kann auch mehrfach wiederholt werden, wenn dickere Schichten erwünscht sind. Der besondere Vorteil der dünneren Schichten ist die Transparenz für sichtbares Licht. So liefern dünne Zinkoxidbeschichtungen für optische Geräte wie Linsen, insbesondere auch Brillengläser, einen unsichtbaren UV-Breitbandfilter, der für sichtbares Licht zu nahezu 100% durchlässig ist und daher nicht zu Farbverschiebungen führt.

Nach einer bevorzugten Ausführungsform des erfindungsgemäßen Verfahrens zur Oberflächenbeschichtung wird der Zinkoxiddispersion vor dem Auftragen auf die zu beschichtende Oberfläche ein Dotierungsmittel zugesetzt. Als Dotierungsmittel für Zinkoxid eignen sich insbesondere Metallionen mit einem Elektron mehr oder einem Elektron weniger auf der äußeren Schale. Besonders geeignet sind Haupt- und Nebengruppenmetalle in der Oxidationsstufe +III. Ganz besonders bevorzugt sind Bor(III), Aluminium(III), Gallium(III) und Indium(III). Diese Metalle können in Form löslicher Salze der Dispersion zugesetzt werden, wobei die Wahl des Metallsalzes danach gerichtet ist, ob es sich im Dispergierungsmittel in gewünschter Konzentration löst. Bei wäßrigen Dispersionen kommen hierbei viele anorganische Salze oder auch Komplexe in Betracht, wie Carbonate, Halogenide, Salze mit EDTA, Nitrate, Salze mit EDTA, Acetylacetonat usw. Eine Dotierung mit Edelmetallen wie Palladium, Platin, Gold etc. ist ebenfalls möglich. Die Donatorenkonzentration kann bis zu 5% betragen. Dotierte Zinkoxidbeschichtungen eignen sich vorzugsweise zum Einsatz als transparente Elektroden für Liquid Crystal Displays, Flat Panel Displays, elektrochrome Fenster (schaltbare Lichtdurchlässigkeit), Photovoltaik-Solarzellen oder beheizbare Spiegel.

Gemäß einer besonders bevorzugten Ausführungsform des erfindungsgemäßen Oberflächenbeschichtungsverfahrens wird die Zinkoxiddispersion über einen spin-coater auf das zu beschichtende Substrat aufgebracht. Dazu wird das zu beschichtende Werkstück in den Spin-Coater eingespannt, auf eine bestimmte Startdrehzahl gebracht und die Zinkoxiddispersion auf das Substrat gegeben. Alternativ kann die Dispersion auch unmittelbar vor oder exakt mit dem Einschalten des Spin-Coater auf das Substrat gebracht werden. Der Spin-Coater vollzieht dann im weiteren ein Drehzahlprogramm, das individuell auf das zu beschichtende Werkstück abzustimmen ist. Dies ist ein in der Technik üblicherweise notwendiger Anpassungsprozeß, der sich nach der Oberflächenbeschaffenheit des Substrats (Rauhigkeit, Benetzbarkeit, Größe, usw.) sowie nach den Eigenschaften des Beschichtungsmittels richtet (Viskosität, Benetzung, Dichte usw.). Die Umdrehungsgeschwindigkeiten bewegen sich üblicherweise im Bereich zwischen einigen hundert bis hin zu einigen tausend Umdrehungen pro Minute. Dabei kann durch eine Programmsteuerung die Drehzahl während des Aufschleuderns ("Auf-spinnes") variiert werden, wenn dies zu einer besseren Beschichtung führt. So kann für den Fall, daß die Dispersion auf das ruhende Substrat aufgebracht wird die Drehzahl nach Einschalten des Spin-Coater langsam gesteigert werden um eine gleichmäßigere Verteilung des Beschichtungsmittels zu erreichen.

Durch das Aufbringen der Schicht über einen Spin-Coater wird gleichzeitig ein Teil des Dispergierungsmittels durch Verdunstung entfernt; dies kann durch gleichzeitiges Anlegen von Vakuum während des Auf-spinnens noch verstärkt werden. Die Schichten, die mit Hilfe dieses Verfahrens in einem Aufschleudervorgang erzeugt werden weisen eine Dicke von etwa 20 bis 300 nm auf. Die Dicke kann über den Gehalt an Zinkoxid in der Dispersion und durch die Wahl des Lösemittels/ Dispergierungsmittels beeinflußt werden. Ein hoher Massenprozent-Gehalt an oberflächenmodifiziertem Zinkoxid erhöht die Schichtdicke bei einem Aufschleudervorgang; ebenfalls die Wahl eines Dispergierungsmittels mit einer höheren Viskosität. Diese Schichten zeichnen sich darüber hinaus durch eine sehr gleichmäßige Schichtdicke und geringe Rauhigkeit aus. So sind Schichten herstellbar, die eine Oberflächenrauhigkeit von weniger als 1 nm aufweisen. Durch die durch dieses Verfahren erzielbare geringe Oberflächenrauhigkeit und gleichmäßige Dicke weisen die Beschichtungen sehr homogene optische und elektrische Eigenschaften auf.

Die Herstellung dickerer Schichten in einem Aufschleudervorgang ist jedoch ebenfalls möglich, jedoch sind diese nicht mehr komplett transparent. So können Schichten bis zu 1,2 μm Dicke erzeugt werden. Alternativ zum Spin-Coating kann die Beschichtung jedoch auch durch Tauchung oder Aufsprühen einer Zinkoxiddispersion erreicht werden.

Gemäß einer weiteren besonders bevorzugten Ausführungsform des erfindungsgemäßen Beschichtungsverfahrens wird die bereits beschichtete Oberfläche anschließend auf eine Temperatur zwischen 100°C und 1000°C erwärmt. Dies wird vorzugsweise unter Sauerstoffausschluß oder unter reduzierender Atmophäre ($H_2$ oder $H_2$-haltig) durchgeführt. Die Temperdauer beträgt zwischen 10 Minuten und 6 Stunden. Die Erwärmung ist besonders vorteilhaft, weil damit einerseits Reste des Dispergierungsmittels entfernt werden und andererseits mögliche mechanische Spannungen in der Beschichtung ausheilen können. Werden dotierte Zinkoxidschichten hergestellt, so ist eine Erwärmung

auf eine Temperatur von mehr als 300 °C vorteilhaft, da ab etwa dieser Temperatur ein. Inkorporieren des Dotierungsmittels in das Zinkoxidgitter in relativ kurzer Zeit vonstatten geht. Auch kann es von Vorteil sein, die Oberflächenbeschichtung höheren Temperaturen auszusetzen um bspw. das Dotierungsmittel bzw. das Anion des Dotierungsmittels zu entfernen (z.B. durch Oxidation). Beim Erhitzen unter reduzierender Atmosphäre können beispielsweise Edelmetallprecursor bis zum Metall reduziert werden.

Eine Erwärmung bis in die Nähe des Schmelzpunktes von Zinkoxid kann ebenfalls vorteilhaft sein, um die einzelnen Nanopartikel zu einer kontinuierlichen Schicht zusammenzusintern.

Nach einer weiteren besonders bevorzugten Ausführungsform des erfindungsgemäßen Oberflächenbeschichtungsverfahrens werden über dieses Verfahren elektrisch leitfähige Oberflächen und Schichten hergestellt. Die daraus resultierenden Vorteile wurden bereits weiter oben eingehend erläutert.

Ein weiterer Gegenstand der vorliegenden Erfindung ist ein kosmetisches Mittel, das ein erfindungsgemäß oberflächenmodifiziertes Zinkoxid oder eine Zinkoxiddispersion enthält. Dies ist besonders vorteilhaft, da aufgrund der feinen Verteilung der Zinkoxidpartikel diese ihre hautberuhigende Wirkung effektiver entfalten können. Hierzu wurde ein Vergleichsversuch (Beispiel 3) im Vergleich mit handelsüblichen hautberuhigenden Mitteln durchgeführt. Hierbei zeigt sich deutlich die verbesserte Wirkung im Vergleich zu üblichen hautberuhigenden Wirkstoffen. Ein weiterer Vorteil liegt darin, daß beim Auftragen auf z.B. die Haut aufgrund der geringen Partikelgröße kein Reibeeffekt auftritt, sondern ein sanftes Auftragen möglich ist, was ein angenehmes Hautgefühl hervorruft.

Nach einer weiteren Ausführungsform des kosmetischen Mittels dient dieses der Pflege oder dem Schutz der Haut insbesondere zum Sonnenschutz bzw. zur Pflege bei Sonnenlichtexposition und liegt in Form einer Emulsion, einer Dispersion, einer Suspension, einer wässrigen Tensidzubereitung, einer Milch, einer Lotion, einer Creme, eines Balsams, einer Salbe, eines Gels, eines Granulats, eines Puders, eines Stiftpräparates, wie z.B. eines Lippenstifts, eines Schaums, eines Aerosols oder eines Sprays vor. Solche Formulierungen sind gut geeignet für topische Zubereitungen. Als Emulsionen kommen Öl-in-Wasser-Emulsionen und W/O-Emulsionen oder Mikroemulsionen in Frage. Dies ist besonders vorteilhaft weil durch den Einsatz in Sonneschutzmittel die UVabsorbierende und die hautberuhigende Wirkung des Zinkoxids gleichzeitig genutzt werden können. Darüber hinaus eignet sich das erfindungsgemäß oberflächenmodifizierte Zinkoxid sehr gut zum Einsatz in Sonnenschutzmittel, da die Partikel in einer Größe herstellbar sind, die sie für das menschliche Auge tranparent erscheinen lassen. Dadurch entsteht bei der Anwendung kein weißer Schleier auf der Haut. Ein weiterer Vorteil ist die Tatsache, daß es sich bei Zinkoxid um einen UV-Breitbandfilter handelt, dessen UV-Absorptionsverhalten es erlaubt, ein Sonnenschutzmittel zu schaffen, daß keine weiteren chemischen UV-Filtersubstanzen mehr benötigt. Dadurch kann die Gefahr von Haut-Irritationen oder allergischen Reaktionen durch Zersetzungsprodukte chemischer Filter oder durch diese Substanzen selbst vermieden werden, was die allgemeine Verträglichkeit eines derart gestalteten Sonnenschutzmittels stark erhöht.

Im Regelfall wird das kosmetische Mittel zur topischen Applikation auf der Haut verwendet. Unter topischen Zubereitungen sind dabei solche Zubereitungen zu verstehen, die dazu geeignet sind, die Wirkstoffe in feiner Verteilung und bevorzugt in einer durch die Haut resobierbaren Form auf die Haut aufzubringen. Hierfür eignen sich z.B. wässrige und wässrig-alkoholische Lösungen, Sprays, Schäume, Schaumaerosole, Salben, wässrige Gele, Emulsionen vom O/W- oder W/O-Typ, Mikroemulsionen oder kosmetische Stiftpräparate.

Nach einer bevorzugten Ausführungsform des erfindungsgemäßen kosmetischen Mittels enthält das Mittel einen Träger. Bevorzugt als Träger ist Wasser, ein Gas, eine Wasser-basierte Flüssigkeit, ein Öl, ein Gel, eine Emulsion oder Mikroemulsion, eine Dispersion oder eine Mischung davon. Die genannten Träger zeigen eine gute Hautverträglichkeit. Besonders vorteilhaft für topische Zubereitungen sind wässrige Gele, Emulsionen oder Mikroemulsionen.

Als Emulgatoren können nichtionogene Tenside, zwitterionische Tenside, ampholytische Tenside oder anionische Emulgatoren verwendet werden. Die Emulgatoren können in der erfindungsgemäßen Zusammensetzung in Mengen von 0,1 bis 10, vorzugsweise 1 bis 5, Gew.-%, bezogen auf die Zusammensetzung, enthalten sein.

Als nichtionogenes Tensid kann bspw. ein Tensid aus mindestens einer der folgenden Gruppen verwendet werden:

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- $C_{12/18}$-Fettsäuremono- und -diester von Anlagerungsprodukten von 1 bis 30 Mol Ethylenoxid an Glycerin;
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- Alkylmono- und -oligoglycoside mit 8 bis 22 Kohlenstoffatomen im Alkylrest und deren ethoxylierte Analoga;

- Anlagerungsprodukte von 15 bis 60 Mol Ethylenoxid an Ricinusöl und/oder gehärtetes Ricinusöl;
- Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
- Anlagerungsprodukte von 2 bis 15 Mol Ethylenoxid an Ricinusöl und/oder gehärtetes Ricinusöl;
- Partialester auf Basis linearer, verzweigter, ungesättigter bzw. gesättigter $C_{6/22}$-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
- Polyalkylenglycole
- Betaine
- Esterquats

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- oder eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat.

Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das $C_{12/18}$-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind. Des weiteren können als anionische Emulgatoren Alkylethersulfate, Monoglyceridsulfate, Fettsäuresulfate, Sulfosuccinate und/oder Ethercarbonsäuren eingesetzt werden.

Als Ölkörper kommen Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen $C_6$-$C_{22}$-Fettalkoholen, Ester von verzweigten $C_6$-$C_{13}$-Carbonsäuren mit linearen $C_6$-$C_{22}$-Fettalkoholen, Ester von linearen $C_6$-$C_{22}$-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare $C_6$-$C_{22}$-Fettalkoholcarbonate, Guerbetcarbonate, Ester der Benzoesäure mit linearen und/oder verzweigten $C_6$-$C_{22}$-Alkoholen (z.B. Finsolv® TN), Dialkylether, Ringöffnungsprodukte von epoxidierten Fettsäureestern mit Polyolen, Siliconöle

und/oder aliphatische bzw. naphthenische Kohlenwasserstoffe in Betracht. Als Ölkörper können ferner auch Siliconverbindungen eingesetzt werden, beispielsweise Dimethylpolysiloxane, Methylphenylpolysiloxane, cyclische Silicone sowie amino-, fettsäure-, alkohol-, polyether-, epoxy-, fluor-, alkyl- und/oder glykosidmodifizierte Siliconverbindungen, die bei Raumtemperatur sowohl flüssig als auch harzförmig vorliegen können. Die Ölkörper können in den erfindungsgemäßen Mitteln in Mengen von 1 bis 90, vorzugsweise 5 bis 80, und insbesondere 10 bis 50, Gew.-% - bezogen auf die Zusammensetzung enthalten sein.

Nach einer besonders bevorzugten Ausführungsform enthält die erfindungsgemäße Zusammensetzung weitere UV-Lichtschutzfilter in Form löslicher Verbindungen oder anderer Pigmente.

Obwohl es wie bereits weiter oben beschrieben möglich ist, mit Hilfe der erfindungsgemäßen Zinkoxidpartikel ein Sonnenschutzmittel zu schaffen, das gute UV-Absorptionseigenschaften ohne weitere UV-Filtersubstanzen erreicht, kann es im Einzelfall gewünscht sein, dem kosmetischen Mittel bzw. dem Sonnenschutzmittel weitere UV-Filtersubstanzen zuzusetzen. Dies kann z.B. dann erforderlich sein, wenn ein besonderer Schwerpunkt bei der Filterleistung gelegt werden soll. Der erfindungsgemäßen Zusammensetzung können ein oder mehrere weitere UV-Lichtschutzfilter zugesetzt werden.

Im Falle der löslichen Verbindungen sind unter UV-Lichtschutzfiltern organische Substanzen zu verstehen, die in der Lage sind, ultraviolette Strahlen zu absorbieren und die aufgenommene Energie in Form längerwelliger Strahlung, z.B. Wärme, wieder abzugeben. Die organischen Substanzen können öllöslich oder wasserlöslich sein.

Als öllösliche UV-B-Filter können z.B. folgende Substanzen verwendet werden:

- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino) benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester, 4-Methoxyzimtsäureisopentylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin (Octyltriazone) und Dioctyl Butamido Triazon (Uvasorb® HEB).
- Propan-1,3-dione, wie z.B. 1-(4-tert.Butylphenyl)-3-(4'methoxyphenyl)propan-1,3-dion.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B.. 4-(2-Oxo-3-bornylidenmethyl)benzolsulfonsäure und 2-Methyl-5-(2-oxo-3-bornyliden)sulfonsäure und deren Salze.

Besonders bevorzugt ist die Verwendung von Estern der Zimtsäure, vorzugsweise 4-Methoxyzimtsäure-2-ethylhexylester, 4-Methoxyzimtsäureiso-pentylester, 2-Cyano-3-phenyl-zimtsäure-2-ethylhexylester (Octocrylene). Des weiteren ist die Verwendung von Derivaten des Benzophenons, insbesondere 2-Hydroxy-4-methoxybenzophenon, 2-Hydroxy-4-methoxy-4'-methylbenzophenon, 2,2'-Dihydroxy-4-methoxybenzophenon sowie der Einsatz von Propan-1,3-dionen, wie z.B. 1-(4-tert.Butylphenyl)-3-(4'methoxyphenyl)propan-1,3-dion bevorzugt.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan oder 1-Phenyl-3-(4'-isopropylphenyl)-propan-1,3-dion. Die UV-A und UV-B-Filter können selbstverständlich auch in Mischungen eingesetzt werden.

Als weitere Lichtschutzfilter können aber auch andere unlösliche Pigmente, z.B. feindisperse Metalloxide bzw. Salze wie beispielsweise Titandioxid, Eisenoxid, Aluminiumoxid, Ceroxid, Zirkoniumoxid, Silicate (Talk), Bariumsulfat und Zinkstearat verwendet werden. Die Partikel sollten dabei einen mittleren Durchmesser von weniger als 100 nm, vorzugsweise zwischen 5 und 50 nm und insbesondere zwischen 15 und 30 nm aufweisen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Der Gesamtanteil der Lichtschutzmittel in dem erfindungsgemäßen Sonnenschutzmittel liegt üblicherweise bei 1 bis 20, vorzugsweise 5 bis 15 Gew.-%. Die erfindungsgemäße Zusammensetzung als solche kann 1 bis 95, vorzugsweise 5 bis 80, und insbesondere 10 bis 60 Gew.-% Wasser enthalten.

Nach einer besonders bevorzugten Ausführungsform enthält die erfindungsgemäße kosmetische Zusammensetzung ferner pflegende Substanzen, weitere kosmetische Wirkstoffe und/oder Hilfs- und Zusatzstoffe. Als weitere kosmetische Wirkstoffe werden insbesondere Hautfeuchthaltemittel, antimikrobielle Stoffe und/oder deodorierende oder schweißhemmende Stoffe eingesetzt. Dies hat den Vorteil, dass sich weitere gewünschte Effekte erzielen lassen, die zur Pflege oder Behandlung der Haut beitragen oder beispielsweise das Wohlempfinden des Anwenders der kosmetischen Zusammensetzung bei der Verwendung dieser Zusammensetzung steigern.

So können in der kosmetischen Zusammensetzung neben dem Träger, dem oberflächenmodifizierten Zinkoxid, Wasser und physiologisch geeigneten Lösemitteln unter anderem auch pflegende Bestandteile, wie z.B. Öle, Wachse, Fette, rückfettende Substanzen, Verdickungsmittel, Emulgatoren und Duftstoffe enthalten sein. Ein hoher Anteil an pflegenden Substanzen ist insbesondere zur topischen prophylaktischen oder kosmetischen Behandlung der Haut vorteilhaft. Besonders vorteilhaft ist es, wenn die Zusammensetzung neben den in vielen Fällen ebenfalls Pflegewirkung aufweisenden tierischen und pflanzlichen Fetten und Ölen noch weitere Pflegekomponenten enthält. Die Gruppe der pflegenden Wirkstoffe, die zum Einsatz kommen können, umfasst z.B.: Fettalkohole mit 8-22 C-Atomen, insbesondere Fettalkohole von natürlichen Fettsäuren; tierische und pflanzliche Proteinhydrolysate, insbesondere Elastin-, Kollagen-, Keratin-, Milcheiweiß-, Sojaprotein-, Seidenprotein-, Haferprotein-, Erbsenprotein-, Mandelprotein- und Weizenproteinhydrolysate; Vitamine und Vitaminvorstufen, insbesondere die der Vitamin-Gruppen A und B; Mono-, Di- und Oligosaccharide; Pflanzenextrakte; Honigextrakte; Ceramide; Phospholipide; Vaseline, Paraffin- und Silikonöle; Fettsäure- und Fettalkoholester, insbesondere die Monoester der Fettsäuren mit Alkoholen mit 3 - 24 C-Atomen.

Zu den in der erfindungsgemäßen Zusammensetzung bevorzugt einzusetzenden Vitaminen, Provitaminen oder Vitaminvorstufen gehören unter anderem:

- Vitamine, Provitamine und Vitaminvorstufen aus den Gruppen A, C, E und F, insbesondere 3,4-Didehydroretinol (Vitamin $A_2$), β-Carotin (Provitamin des Vitamin $A_1$), Ascorbinsäure (Vitamin C), sowie die Palmitinsäureester, Glucoside oder Phosphate der Ascorbinsäure, Tocopherole, insbesondere α-Tocopherol sowie seine Ester, z. B. das Acetat, das Nicotinat, das Phosphat und das Succinat; weiterhin Vitamin F, worunter essentielle Fettsäuren, besonders Linolsäure, Linolensäure und Arachidonsäure, verstanden werden; Vitamin A und seine Derivate und Provitamine zeigen vorteilhafterweise einen besonderen hautglättenden Effekt.

Zu den in der erfindungsgemäßen Zusammensetzung bevorzugt einzusetzenden Vitaminen, Provitaminen oder Vitaminvorstufen der Vitamin B-Gruppe oder deren Derivaten sowie den Derivaten von 2-Furanon gehören unter anderem:

- Vitamin $B_1$, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.

- Vitamin $B_2$, Trivialname Riboflavin, chemische Bezeichung 7,8-Dimethyl-10-(1-D-ribityl)-benzo[g]pteridin-2,4(3*H*, 10*H*)-dion. In freier Form kommt Riboflavin z. B. in Molke vor, andere Riboflavin-Derivate lassen sich aus Bakterien und Hefen isolieren. Ein erfindungsgemäß ebenfalls geeignetes Stereoisomeres des Riboflavin ist das aus Fischmehl oder Leber isolierbare Lyxoflavin, das statt des D-Ribityl einen D-Arabityl-Rest trägt. Bevorzugt werden Riboflavin oder seine Derivate in Mengen von 0,05 bis 1 Gew.-%, bezogen auf das gesamte Mittel, eingesetzt.

- Vitamin $B_3$. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.

- Vitamin $B_5$ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden.

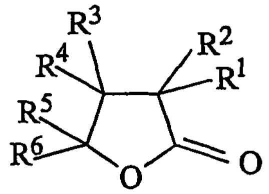

(I)

Bevorzugt sind die 2-Furanon-Derivate, in denen die Substituenten $R^1$ bis $R^6$ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten $C_2$-$C_4$-Kohlenwasserstoffrest, einen gesättigten oder ein- oder zweifach ungesättigten, verzweigten oder linearen Mono-, Di- oder Trihydroxy-$C_2$-$C_4$-Kohlenwasserstoffrest oder einen gesättigten oder ein- oder zweifach ungesättigten, verzweigten oder linearen Mono-, Di- oder TriaminO-$C_2$-$C_4$-Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) $R^1$ für eine Hydroxylgruppe, $R^2$ für ein Wasserstoffatom, $R^3$ und $R^4$ für eine Methylgruppe und $R^5$ und $R^6$ für ein Wasserstoffatom stehen. Das Stereoisomer (R)-Pantolacton entsteht beim Abbau von Pantothensäure.

Vorteilhafterweise verleihen diese Verbindungen der erfindungsgemäßen kosmetischen Zusammensetzung feuchtigkeitsspendende sowie hautberuhigende Eigenschaften.

Die genannten Verbindungen des Vitamin $B_5$-Typs sowie die 2-Furanonderivate sind in den erfindungsgemäßen Mitteln bevorzugt in einer Gesamtmenge von 0,05 bis 10 Gew.-%, bezogen auf das gesamte Mittel, enthalten. Gesamtmengen von 0,1 bis 5 Gew.-% sind besonders bevorzugt.

- Vitamin $B_6$, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin $B_6$ ist in den erfindungsgemäßen Mitteln bevorzugt in Mengen von 0,0001 bis 1,0 Gew.-%, insbesondere in Mengen von 0,001 bis 0,01 Gew.-%, enthalten.

- Vitamin $B_7$ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Panthenol, Pantolacton, Nicotinsäureamid sowie Biotin sind erfindungsgemäß ganz besonders bevorzugt.

Unter Hilfs- und Zusatzstoffen sind Stoffe zu verstehen, die zur Verbesserung der ästhetischen, anwendungstechnischen und/oder kosmetischen Eigenschaften geeignet sind, wie z.B. Co-Emulgatoren, organische Lösemittel, Überfettungsmittel, Stabilisatoren, Antioxidationsmittel, Wachse oder Fette, Konsistenzgeber, Verdickungsmittel, Bräunungsmittel, Vitamine, kationische Polymere, biogene Wirkstoffe, Konservierungsmittel, Hydrotope, Solubilisatoren, Farb- und Duftstoffe.

Beispielsweise können folgende Hilfs- und Zusatzstoffe verwendet werden:

- Allantoin,

- Aloe Vera,
- Bisabolol,
- Ceramide und Pseudoceramide,
- Antioxidationsmittel verbessern vorteilhafterweise die Stabilität der erfindungsgemäßen Zusammensetzungen. Antioxidantien sind beispielsweise Aminosäuren (z. B. Glycin, Histidin, Tyrosin, Tryptophan) und deren Derivate, Imidazol und Imidazolderivate (z. B. Urocaninsäure), Peptide wie z. B. D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis μmol/kg), ferner Metallchelatoren (z. B. α-Hydroxyfettsäuren, EDTA, EGTA, Phytinsäure, Lactoferrin), α-Hydroxysäuren (z. B. Zitronensäure, Milchsäure, Äpfelsäure), Huminsäuren, Gallensäure, Gallenextrakte, Gallussäureester (z. B. Propyl-, Octyl- und Dodecylgallat), Flavonoide, Catechine, Bilirubin, Biliverdin und deren Derivate, ungesättigte Fettsäuren und deren Derivate (z. B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, -dipalmitat, -acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate (z. B. Ferulasäure, Ethylferulat, Kaffeesäure), Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und Zink-Derivate (z. B. ZnO, $ZnSO_4$), Selen und Selen-Derivate (z. B. Selenmethionin), Stilbene und Stilben-Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z. B. Teebaumöl, Rosmarinextrakt und Rosmarinsäure), die diese Antioxidantien enthalten, eingesetzt werden. Als lipophile, öllösliche Antioxidantien aus dieser Gruppe sind Tocopherol und dessen Derivate, Gallussäureester, Flavonoide und Carotinoide sowie Butylhydroxytoluol/anisol bevorzugt. Als wasserlösliche Antioxidantien sind Aminosäuren, z. B. Tyrosin und Cystein und deren Derivate sowie Gerbstoffe, insbesondere solche pflanzlichen Ursprungs bevorzugt. Die Gesamtmenge der Antioxidantien in den erfindungsgemäßen kosmetischen Zusammensetzungen beträgt 0,001 - 20 Gew.-%, vorzugsweise 0,05 - 10 Gew.-%, insbesondere 0,1 - 5 Gew.-% und ganz besonders bevorzugt 0,1 bis 2 Gew.-%.
- Triterpene, insbesondere Triterpensäuren wie Ursolsäure, Rosmarinsäure, Betulinsäure, Boswelliasäure und Bryonolsäure,
- Monomere Catechine, besonders Catechin und Epicatechin, Leukoanthocyanidine, Catechinpolymere (Catechin-Gerbstoffe) sowie Gallotannine,
- Verdickungsmittel, z. B. Gelatine, Pflanzengumme wie Agar-Agar, Guar-Gum, Alginate, Xanthan-Gum, Gummi arabicum, Karaya-Gummi oder Johannisbrotkernmehl, natürliche und synthetische Tone und Schichtsilikate, z. B. Bentonit, Hectorit, Montmorillonit oder Laponite® , vollsynthetische Hydrokolloide wie z. B. Polyvinylalkohol, und außerdem Ca-, Mg- oder Zn-Seifen von Fettsäuren,
- Pflanzenglycoside,
- Strukturanten wie Maleinsäure und Milchsäure,
- Dimethylisosorbid,
- Alpha-, beta- sowie gamma-Cyclodextrine, insbesondere zur Stabilisierung von Retinol,

- Lösemittel, Quell- und Penetrationsstoffe wie Ethanol, Isopropanol, Ethylenglykol, Propylenglykol, Propylenglykolmonoethylether, Glycerin und Diethylenglykol, Carbonate, Hydrogencarbonate, Guanidine, Harnstoffe sowie primäre, sekundäre und tertiäre Phosphate
- Parfümöle, Pigmente sowie Farbstoffe zum Anfärben des Mittels,
- Substanzen zur Einstellung des pH-Wertes, z. B. α- und β-Hydroxycarbonsäuren,
- Komplexbildner wie EDTA, NTA, β-Alanindiessigsäure und Phosphonsäuren,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Treibmittel wie Propan-Butan-Gemische, $N_2O$, Dimethylether, $CO_2$ und Luft.

Die Zugabe von Allantoin, Bisabolol und/oder Aloe Vera auch in Form von Extrakten zu den erfindungsgemäßen kosmetischen Zusammensetzungen verbessert die weiterhin die hautberuhigenden, feuchtigkeitsspendenden und hautpflegende Eigenschaften der Formulierungen und ist daher besonders bevorzugt.

Als weitere Inhaltsstoffe kann die erfindungsgemäße kosmetische Zusammensetzung in untergeordneten Mengen weitere, mit den anderen Inhaltsstoffen kompatible Tenside enthalten. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid-(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutarnate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine.

Nach einer weiteren besonders bevorzugten Ausführungsform wird das erfindungsgemäße kosmetische Mittel als Sonnenschutzmittel verwendet. Die daraus resultierenden Vorteile wurden bereits eingehend erläutert. In Beispiel 5 ist eine Basisrezeptur mit Einsatz des erfindungsgemäß beschichteten Zinkoxids (mit Polyglycoldisäure) beschrieben.

Der Einsatz der erfindungsgemäßen Zinkoxiddispersionen ist insbesondere ebenfalls möglich in Haarkosmetika wie Shampoos, Conditioner, Spülungen, Haarwässer, Haargel, Haarspray etc.. Insbesondere leave-on Produkte, die nach erfolgter Applikation auf dem Haar bzw. der Kopfhaut verbleiben sind besonders gut geeignet. Das so auf die Kopfhaut und das Haar aufgetragene Zinkoxid kann somit auch dort als UV-Schutzmittel wirken bzw. auf der Kopfhaut seine hautberuhigende Wirkung entfalten.

Nach einer bevorzugten Ausführungsform des erfindungsgemäßen kosmetischen Mittels wird das kosmetische Mittel auf die Oberfläche des zu behandelnden bzw. zu schützenden Körpers also topisch aufgetragen. Diese Applikationsform ist besonders vorteilhaft, da sie einfach zu handhaben ist, so dass Fehldosierungen weitestgehend ausgeschlossen sind. Ferner lässt sich ein zusätzlicher pflegender Effekt für die Haut erreichen. Falls nur einzelne Körperteile der Sonnenstrahlung ausgesetzt werden, kann das Sonnenschutzmittel außerdem nur gezielt auf diese Körperteile aufgetragen werden.

Ein weiterer Gegenstand der vorliegenden Erfindung ist die Verwendung von erfindungsgemäß oberflächenmodiziertem Zinkoxid oder Zinkoxiddispersionen zum UV-Schutz. Dies ist besonders vorteilhaft, da aufgrund der Feinteiligkeit des oberflächenmodifizierten Zinkoxids und der guten Verteilung eine besonders hohe UV-Absorption erreicht wird.

Ein weiterer Gegenstand der vorliegenden Erfindung ist die Verwendung von erfindungsgemäß oberflächenmodiziertem Zinkoxid oder Zinkoxiddispersionen als antimikrobieller Wirkstoff. Die Verwendung dieser Partikel ist für

diesen Einsatzzweck besonders vorteilhaft, da aufgrund der Feinteiligkeit der Partikel und der daraus resultierenden großen Oberfläche, die antimikrobielle Wirkung stark verbessert ist und andererseits aufgrund der guten Dispergiereigenschaften des Materials das Zinkoxid in fein verteilter Form vorliegt. Somit kann das Zinkoxid problemlos in verschiedenen Darreichungsformen eingesetzt werden wie beispielsweise Cremes, Hautmilch, Lotionen oder Tonics.

Ein weiterer Gegenstand der vorliegenden Erfindung ist ein pharmazeutisches Mittel, das ein oberflächenmodifiziertes Zinkoxid oder eine Zinkoxiddispersion enthält. Dieses pharmazeutische Mittel weist sich dadurch aus, daß aufgrund der Feinteiligkeit der Partikel die pharmazeutische Wirksamkeit stark erhöht ist. Dies wird aus den Ergebnissen von Beispiel 3, sowie den Abbildungen 1 und 2 deutlich. Darüber hinaus besitzt das erfindungsgemäße pharmazeutische Mittel den Vorteil, daß aufgrund der bereits oben beschriebenen guten Langzeitstabilität der Zinkoxiddispersionen auf den Zusatz von Stabilisatoren verzichtet werden kann, die eine Entmischung verhindern. Somit wird zusätzlich die Verträglichkeit des pharmazeutischen Mittels erhöht.

**Beispiel 1:**

In einem 250 ml Rundkolben werden 6,2 g ZnO in 80 ml THF suspendiert. Zu dieser Dispersion werden 1,5g Polyethylenglycoldisäure600 (Aldrich Prod. Nr. 40,703-8, Ch.Nr. 24441-030), gelöst in 40 ml THF, getropft. Danach wird die Mischung 30 Minuten unter Rückfluß gekocht. Anschließend wird auf Raumtemperatur abgekühlt und das THF abdekantiert. Der Feststoff wird einmal mit 100 ml THF versetzt und 15 Minuten gerührt. Das THF wird erneut abdekantiert und das modifizierte ZnO im Vakuum getrocknet. Das so erhaltene oberflächenmodifizierte ZnO kann ohne Nachbehandlung in Wasser dispergiert werden.

Nach Bestimmung der Partikelgrößenverteilung mittels dynamischer Lichtstreuung sind 95% der Partikel kleiner als 50 nm und die volumengemittelte Partikelgröße liegt bei 27 nm.

**Beispiel 2:**

In einem 500 ml Dreihalskolben werden 3,5 g (0,043 mol) ZnO in 100 ml THF suspendiert. Zu dieser Suspension wird eine Lösung von 2 g 2-[2-(2-Methoxyethoxy)-ethoxy]-essigsäure (Fluka Art.Nr. 64732, Ch.Nr. RB13802) in 30 ml THF gegeben. Die Reaktionslösung wird zum Sieden erhitzt und 1 Std. bei der Temperatur gehalten. In dieser Zeit bildet sich eine nur wenig trübe Dispersion. Nach dem Abkühlen auf Raumtemperatur wird das Lösemittel abdestilliert. Der resultierende Feststoff wird in 100 ml Wasser dispergiert und die überschüssige Säure durch Dialyse abgetrennt. Nach dem Abdestillieren des Wassers unter vermindertem Druck, wird der erhaltene Feststoff im Vakuum getrocknet.

Das so erhaltene, modifizierte ZnO kann in Wasser und außerdem im Methanol, Ethanol, Isopropanol, Aceton, Toluol ohne Nachbehandlung auf Primärpartikelgröße dispergiert werden.

Nach der Bestimmung der Partikelgrößenverteilung über XRD sind 90% der Partikel kleiner als 22 nm und die mittlere volumengewichtete Partikelgröße liegt bei etwa 14 nm.

**Beispiel 3:**

Bei diesem Vergleichsversuch wurde die hautberuhigende Wirkung der erfindungsgemäßen oberflächenmodifizierten Zinkoxidpartikel/-dispersionen im Vergleich mit anderen hautberuhigenden Wirkstoffen untersucht. Hierzu wurden folgende Substanzen eingesetzt:

1. Mit Polyethylenglykol-di-essigsäure oberflächenmodifiziertes Zinkoxid, in 0,5%iger Dispersion in Wasser;
2. Mit Polyethylenglykol-di-essigsäure oberflächenmodifiziertes Zinkoxid, 2%ige Dispersion in Wasser;
3. Ohne Hautreizung - gespült mit Wasser
4. Mit SDS-Reizung - gespült mit Wasser
5. Mit SDS-Reizung - mit einer Cortison-Salbe behandelt
6. D-Pantenol, 0,5%ig
7. D-Pantenol, 2%ig
8. Zinkoxiddispersion nach DE 19907704, 0,5%ig

9. Zinkoxid nach DE 19907704, 2%ig

10.Lösemittel aus (8) und (9) gemäß DE 19907704: Ethylenglycol/Wasser/Triethanolamin

Die oben genannten Rezepturen 1 und 2 entsprechen den erfindungsgemäßen Zinkoxiddispersionen. Diese Rezepturen wurden hinsichtlich ihrer hautberuhigenden Wirkung in-vitro an einem Epidermismodell getestet und mit der Wirkung von D-Pantenol in zwei verschiedenen Konzentrationen (Rezepturen 6 und 7), sowie mit einer gemäß DE 19907704 hergestellten Zinkoxiddispersion (Rezepturen 8 und 9) verglichen.

Als Epidermismodell wurde das 3-dimensionale Modell EPIDERM® (Firma Martek, USA) verwendet. Die Modelle wurden zur Auslösung einer Hautreizung mit einem Tensid (0,16% SDS=Natrium-Dodecyl-Sulfat) über eine 1 Std. inkubiert. Nach Abspülen des SDS erfolgte eine Behandlung mit den Prüfsubstanzen. Als interne Positivkontrolle wurde Cortisoncreme mitgeführt, welches entsprechend nach SDS-Reizung aufgetragen wurde. Für die Tensidkontrolle wurden die Modelle nach SDS-Schädigung mit Aqua demin. bzw. Lösemittel anstelle der Prüfsubstanzen behandelt, für die Negativkontrolle erfolgte die Behandlung lediglich mit Aqua demin. anstelle von Tensid- bzw. Prüfsubstanz. Es erfolgte eine weitere Inkubationszeit von 20 Std., nach der die Vitalität der Modelle im MTT-Test (Journal of immunological Methods, 65 (1983) 55 - 63) überprüft und die Ausschüttung des Entzündungsmediators IL-1$\alpha$ mittels ELISA (Enzyme linked immuno sorbent assay) bestimmt wurde. Die Ergebnisse sind in Abbildung 1 dargestellt. Hierbei bedeutet eine hohe Ausschüttung an Interleucin-1$\alpha$ eine geringe Reduzierung der Hautreizung und eine niedrige Ausschüttung eine gute hautberuhigende Wirkung. In diesem Vergleichstest zeigen die erfindungsgemäßen Zinkoxiddispersionen eine sehr gute hautberuhigende Wirkung. Rezepturen 8 und 9 liefern keine verläßlichen Ergebnisse bezüglich ihrer hautberuhigenden Wirkung, da das in der Beschreibung der DE 19907704 angegebene Lösemittel bereits zu einer starken Schädigung der Zellen im Hautmodell führte. Dies kann aus Abbildung 2 entnommen werden, die die Ergebnisse der Vitalitätsbestimmung gemäß MTT-Test zusammenfaßt.

**Beispiel 4:**

Herstellung einer Zinkoxid-Schicht auf einem Glassubstrat.

Auf einen runden Glaswafer (Corning 1737 F) werden 500 $\mu$l einer 20% igen Dispersion von mit Polyethylenglycoldisäure600 oberflächenmodifiziertem Zinkoxid in Ethanol mit dem Einschalten des Spin-Coaters gleichmäßig aufgebracht. Das Drehzahlprogramm ist derart eingestellt, daß die Drehzahl 5 s bei 500 min$^{-1}$ verweilt, während die Dispersion auf das Substrat gespritzt wird. Danach wird die Drehzahl über ein Intervall von 10 s auf 4000 min$^{-1}$ erhöht und diese Drehzahl für 20 s beibehalten. Anschließend wird wieder auf 0 min$^{-1}$ abgebremst.

Danach wird das beschichtete Substrat im Muffelofen bei 350°C für eine Stunde in Luft-Atmosphäre getempert (dT/dt=1 °C/min)

Die Schichtdicke wird mit Hilfe eines Profilometers zu 200 nm bestimmt. Die Auswertung der mittleren Teilchengröße über die statistische Auswertung einer REM-Aufnahme liefert einen Wert 30 nm. Die Transmission liegt im visuellen Bereich (400-800 nm) bei 98,9%. Der spezifische elektrische Widerstand wurde über eine 4-Punkt Messung zu 1,1*10$^4$ □cm bestimmt.

**Beispiel 5:**

Die folgenden Komponenten wurden miteinander zu einem Sonneschutzmittel verarbeitet:

| Bestandteil | Gew.% |
|---|---|
| Cetiol OE (Di-n-octylether) | 10 |
| Cetiol S (Diisooctylcyclohexan) | 10 |
| Lanette O (Cetylsearylalkohol) | 4,5 |
| Eumulgin B2 (Polyoxyethylen-20-Cetylstearylalkohol | 2 |
| Monomuls 60-35 C (Monoglycerid der Palmfettsäure, gehärtet) | 2 |
| Baysilon M 350 (Silikonöl) | 0,5 |
| Phenonip (Konservierungsmittel) | 1 |
| Zinkoxid (mit Isostearinsäure oberflächenmodifiziert) | 8 |
| Zinkoxid (mit Polyglykoldisäure oberflächenmodifiziert) | 8 |

Tabelle fortgesetzt

| Bestandteil | Gew.% |
|---|---|
| Destilliertes Wasser | Ad 100 |

Herstellung Komponente A:

Die zur Herstellung eingesetzte Apparatur besteht aus einem Becherglas, Propellerrührer und einem Silikonbad mit Heizplatte. Die Emulgatoren und das Baysilon-Öl werden im Becherglas vorgelegt, auf 85-90°C aufgeheizt. Das hydrophobe oberflächenmodifizierte Zinkoxid (mit Isostearinsäuer oberflächenmodifiziert) wird mittels Magnetrührer in einem der kosmetischen Öle oder in einem Gemisch der kosmetischen Öle (Cetiol) dispergiert. Anschließend wird die Zinkoxid-Dispersion zu der Emulgatorschmelze gegeben. Danach wird das auf 85-100°C vorgeheizte Wasser hinzugegeben. Die Mischung wird ca. 10 Minuten bei 85-90°C nachgerührt und anschließend abgekühlt. Während des Abkühlens wurde bei ca. 40°C das Konservierungsmittel hinzugegeben und kräftig untergerührt.

Herstellung einer Sonnencreme

Zur Herstellung der Sonnencreme wird zunächst die oben angegebene Menge an hydrophil oberflächenbeschichtetem Zinkoxid (mit Polyglykoldisäure oberflächenmodifiziert) in der angegebenen Menge Wasser unter Rühren dispergiert und auf 40°C erwärmt. Zu dieser Dispersion wird nun die Komponente A unter kräftigem Rühren hinzugegeben. Anschließend wurde die Creme unter Rühren abgekühlt.

## Patentansprüche

1. Oberflächenmodifiziertes nanopartikuläres Zinkoxid, **dadurch gekennzeichnet, daß** die Oberflächenmodifikation eine Beschichtung mit einer Oligo- oder Polyethylenglycolsäure umfaßt.

2. Oberflächenmodifiziertes Zinkoxid nach Anspruch 1, **dadurch gekennzeichnet, daß** die Oligo- oder Polyethylenglycolsäure der allgemeinen Form $R-CH_2-(O-CH_2-CH_2)_n-O-CH_2-COOH$ entspricht, wobei n eine ganze Zahl von 0 bis 40 ist.

3. Oberflächenmodifiziertes Zinkoxid nach Anspruch 2, **dadurch gekennzeichnet, daß** R einem Rest entspricht, ausgewählt aus H, $CH_3$, $C_2H_5$, $C_3H_7$, $CH(CH_3)_2$, OH, $NH_2$, COOH, $CONH_2$, $CO_2CH_3$, $CO_2C_2H_5$, $CO_2C_3H_7$ und $CO_2$ $CH(CH_3)_2$.

4. Oberflächenmodifiziertes Zinkoxid nach einem der vorstehenden Ansprüche, **dadurch gekennzeichnet, daß** die Oberfläche mit einer Oligo- bzw. Polyethylenglycol-di-essigsäure der allgemeinen Formel $HOOC-CH_2-(O-CH_{2-}CH_2)_n-O-CH_2-COOH$ modifiziert ist und n einer ganzen Zahl von 0 bis 40 entspricht.

5. Oberflächenmodifiziertes Zinkoxid nach Anspruch 2, **dadurch gekennzeichnet, daß** die Oberfläche der Zinkoxidpartikel mit Polyethylenglycoldisäure600 modifiziert ist.

6. Oberflächenmodifiziertes Zinkoxid nach Anspruch 2, **dadurch gekennzeichnet, daß** die Oberfläche mit 2-[2-(2-Methoxyethoxy)-ethoxy]-essigsäure modifiziert ist.

7. Oberflächenmodifiziertes Zinkoxid nach einem der vorstehenden Ansprüche, **dadurch gekennzeichnet, daß** die Zinkoxid-Primärteilchen einen Durchmesser von 1-200 nm besitzen.

8. Oberflächenmodifiziertes Zinkoxid nach einem der vorstehenden Ansprüche, **dadurch gekennzeichnet, daß** die Zinkoxid- Primärteilchen einen Durchmesser von 2-50 nm, insbesondere 3-10 nm, aufweisen.

9. Verfahren zur Herstellung von oberflächenmodifiziertem nanopartikulären Zinkoxid, **dadurch gekennzeichnet, daß**

   a) unbehandeltes Zinkoxid in einem polaren Lösemittel suspendiert wird,
   b) danach mit einer Oligo- oder Polyethylenglycolsäure nach einem der Ansprüche 4 bis 9 versetzt und erhitzt wird und

c) das polare Lösemittel entfernt wird.

**10.** Verfahren nach Anspruch 9, **dadurch gekennzeichnet, daß** beim Verfahrensschritt b) das dispergierte unbehandelte Zinkoxid mit einer Oligo- oder Polyethylenglycolsäure der allgemeinen Form $R\text{-}CH_2\text{-}(O\text{-}CH_2\text{-}CH_2)_n\text{-}O\text{-}CH_2\text{-}COOH$, insbesondere mit 2-[2-(2-Methoxyethoxy)-ethoxy]-essigsäure versetzt und erhitzt wird.

**11.** Verfahren nach Anspruch 9 oder 10, **dadurch gekennzeichnet, daß** das Lösemittel durch Verdampfen unter Normaldruck oder Unterdruck, durch Ausfrieren, Gefriertrocknen, Abfiltrieren und anschließendem Trocknen oder Trocknen bei erhöhter Temperatur bei Normaldruck oder bevorzugt bei vermindertem Druck entfernt wird.

**12.** Verfahren zur Herstellung von Zinkoxiddispersionen, **dadurch gekennzeichnet, daß** oberflächenmodifiziertes Zinkoxid nach einem der Ansprüche 1 bis 8 in Wasser, in ein organisches Lösemittel oder in eine Mischung aus einem organischen Lösemittel und Wasser eingebracht und durch ein geeignetes Verfahren dispergiert wird.

**13.** Verfahren nach Anspruch 12, **dadurch gekennzeichnet, daß** das organische Lösemittel ein Dipolmoment größer als 0,35 $\mu$/D besitzt.

**14.** Verfahren nach Anspruch 12 oder 13, **dadurch gekennzeichnet, daß** das organische Lösemittel ausgewählt ist, oder eine Mischung ist aus Methanol, Ethanol, n-Propanol, i-Propanol, Aceton, Diethylether, Dimethylsulfoxid, Tetrahydrofuran, Methylenchlorid, Trichlormethan Ethanol, Ethylacetat, Isobutylacetat und/oder Toluol.

**15.** Zinkoxiddispersion, hergestellt nach einem Verfahren nach einem der Ansprüche 12 bis 14, **dadurch gekennzeichnet, daß** die Dispersion einen Gehalt an dispergiertem Zinkoxid von 0,001 bis 50 Gew.-% aufweist.

**16.** Zinkoxiddispersion nach Anspruch 15, **dadurch gekennzeichnet, daß** die Dispersion einen Gehalt an dispergiertem Zinkoxid von 0,1 bis 10 Gew.-%, insbesondere 1 bis 5 Gew.-% aufweist.

**17.** Verfahren zur Oberflächenbeschichtung, **dadurch gekennzeichnet, daß** man eine Zinkoxiddispersion, die nach einem Verfahren nach einem der Ansprüche 12 bis 16 hergestellt worden ist auf die zu beschichtende Oberfläche aufträgt und anschließend das Lösemittel entfernt.

**18.** Verfahren nach Anspruch 17, **dadurch gekennzeichnet, daß** der Zinkoxiddispersion vor dem Auftragen auf die zu beschichtende Oberfläche ein Dotierungsmittel zugesetzt wird.

**19.** Verfahren nach Anspruch 17 oder 18, **dadurch gekennzeichnet, daß** die Zinkoxiddispersion in einem Spin-Coater auf das zu beschichtende Substrat aufgebracht wird.

**20.** Verfahren nach Anspruch einem der Ansprüche 17 bis 19, **dadurch gekennzeichnet, daß** die beschichtete Oberfläche anschließend auf eine Temperatur zwischen 100 und 1000°C erwärmt wird, vorzugsweise unter Sauerstoffausschluß oder unter reduzierender Atmosphäre.

**21.** Verfahren nach einem der Ansprüche 17 bis 20 zur Herstellung elektrisch leitfähiger Oberflächen und Schichten.

**22.** Kosmetisches Mittel, enthaltend ein oberflächenmodifiziertes Zinkoxid nach einem der Ansprüche 1 bis 8 oder eine Zinkoxiddispersion nach einem der Ansprüche 15 oder 16.

**23.** Kosmetisches Mittel nach Anspruch 22 zur Pflege oder zum Schutz der Haut, insbesondere zum Sonnenschutz, **dadurch gekennzeichnet, dass** die Zusammensetzung in Form einer Emulsion, einer Dispersion, einer Suspension, einer wäßrigen Tensidzubereitung, einer Milch, einer Lotion, einer Creme, eines Balsams, einer Salbe, eines Gels, eines Granulats, eines Puders, eines Stiftpräparates, eines Schaums, eines Aerosols oder eines Sprays vorliegt.

**24.** Kosmetisches Mittel nach Anspruch 22 oder 23, **dadurch gekennzeichnet, dass** das Mittel weitere UV-Lichtschutzfilter in Form löslicher Verbindungen oder anderer Pigmente enthält.

**25.** Kosmetisches Mittel nach einem der Ansprüche 22 bis 24, **dadurch gekennzeichnet, dass** sie pflegende Substanzen, weitere kosmetische Wirkstoffe, insbesondere Vitamine, Hautfeuchthaltemittel, Antioxidantien, antimikrobielle Wirkstoffe, deodorierende oder schweißhemmende Stoffe, und/oder Hilfs- und Zusatzstoffe enthält.

**26.** Verwendung eines kosmetischen Mittels nach einem der Ansprüche 22 bis 24 als Sonnenschutzmittel.

**27.** Verwendung eines kosmetischen Mittels nach einem der Ansprüche 22 bis 24, **dadurch gekennzeichnet, dass** das kosmetische Mittel topisch angewendet wird.

**28.** Verwendung von oberflächenmodifiziertem Zinkoxid nach einem der Ansprüche 1 bis 8 oder Zinkoxiddispersionen nach einem der Ansprüche 15 oder 16 zum UV-Schutz.

**29.** Verwendung von oberflächenmodifiziertem Zinkoxid nach einem der Ansprüche 1 bis 8 oder Zinkoxiddispersionen nach einem der Ansprüche 15 oder 16 als antimikrobieller Wirkstoff.

**30.** Pharmazeutisches Mittel, enthaltend ein oberflächenmodifiziertes Zinkoxid nach einem der Ansprüche 1 bis 8 oder eine Zinkoxiddispersion nach einem der Ansprüche 15 oder 16.

## Claims

**1.** Surface-modified nanoparticulate zinc oxide, **characterized in that** the surface modification comprises a coating with an oligo- or polyethylene glycolic acid.

**2.** Surface-modified zinc oxide as claimed in claim 1, **characterized in that** the oligo- or polyethylene glycolic acid corresponds to the general formula $R-CH_2-(O-CH_2-CH_2)_n-O-CH_2-COOH$, where n is an integer of 0 to 40.

**3.** Surface-modified zinc oxide as claimed in claim 2, **characterized in that** R is a group selected from H, $CH_3$, $C_2H_5$, $C_3H_7$, $CH(CH_3)_2$, OH, $NH_2$, COOH, $CONH_2$, $CO_2CH_3$, $CO_2C_2H_5$, $CO_2C_3H_7$ and $CO_2CH(CH_3)_2$.

**4.** Surface-modified zinc oxide as claimed in any of the preceding claims, **characterized in that** the surface is modified with an oligo- or polyethylene glycol diacetic acid corresponding to the general formula $HOOC-CH_2-(O-CH_2-CH_2)_n-O-CH_2-COOH$ and n is an integer of 0 to 40.

**5.** Surface-modified zinc oxide as claimed in claim 2, **characterized in that** the surface of the zinc oxide particles is modified with polyethylene glycol diacid 600.

**6.** Surface-modified zinc oxide as claimed in claim 2, **characterized in that** the surface is modified with 2-[2-(2-methoxyethoxy)-ethoxy]-acetic acid.

**7.** Surface-modified zinc oxide as claimed in any of the preceding claims, **characterized in that** the zinc oxide primary particles have a diameter of 1 to 200 nm.

**8.** Surface-modified zinc oxide as claimed in any of the preceding claims, **characterized in that** the zinc oxide primary particles have a diameter of 2 to 50 nm and more particularly 3 to 10 nm.

**9.** A process for the production of surface-modified nanoparticulate zinc oxide, **characterized in that**

a) untreated zinc oxide is suspended in a polar solvent,
b) an oligo- or polyethylene glycolic acid according to any of claims 4 to 9 is added to the suspension which is then heated and
c) the polar solvent is removed.

**10.** A process as claimed in claim 9, **characterized in that**, in step b), an oligo- or polyethylene glycolic acid corresponding to the general formula $R-CH_2-(O-CH_2-CH_2)_n-O-CH_2-COOH$, more particularly 2-[2-(2-methoxyethoxy)-ethoxy]-acetic acid, is added to the dispersed untreated zinc oxide and the suspension is heated.

**11.** A process as claimed in claim 9 or 10, **characterized in that** the solvent is removed by evaporation under normal pressure or reduced pressure, by freezing, freeze-drying, filtering and then drying or drying at elevated temperature under normal pressure or preferably under reduced pressure.

**12.** A process for the production of zinc oxide dispersions, **characterized in that** the surface-modified zinc oxide claimed

in any of claims 1 to 8 is introduced into water, into an organic solvent or into a mixture of an organic solvent and water and dispersed by a suitable method.

**13.** A process as claimed in claim 12, **characterized in that** the organic solvent has a dipole moment of greater than 0.35 μ/D.

**14.** A process as claimed in claim 12 or 13, **characterized in that** the organic solvent is selected from, or is a mixture of, methanol, ethanol, n-propanol, i-propanol, acetone, diethyl ether, dimethyl sulfoxide, tetrahydrofuran, methylene chloride, trichloromethane, ethanol, ethyl acetate, isobutyl acetate and/or toluene.

**15.** A zinc oxide dispersion produced by the process claimed in any of claims 12 to 14, **characterized in that** the dispersion has a content of dispersed zinc oxide of 0.001 to 50% by weight.

**16.** A zinc oxide dispersion as claimed in claim 15, **characterized in that** the dispersion has a content of dispersed zinc oxide of 0.1 to 10% by weight and more particularly 1 to 5% by weight.

**17.** A surface coating process, **characterized in that** a zinc oxide dispersion produced by the process claimed in any of claims 12 to 16 is applied to the surface to be coated and the solvent is subsequently removed.

**18.** A process as claimed in claim 17, **characterized in that** a doping agent is added to the zinc oxide dispersion before application to the surface to be coated.

**19.** A process as claimed in claim 17 or 18, **characterized in that** the zinc oxide dispersion is applied to the substrate to be coated in a spin coater.

**20.** A process as claimed in any of claims 17 to 19, **characterized in that** the coated surface is subsequently heated to a temperature of 100 to 1,000°C, preferably in the absence of oxygen or in a reducing atmosphere.

**21.** A process as claimed in any of claims 17 to 20 for the production of electrically conductive surfaces and layers.

**22.** A cosmetic preparation containing the surface-modified zinc oxide claimed in any of claims 1 to 8 or the zinc oxide dispersion claimed in claim 15 or 16.

**23.** A cosmetic preparation as claimed in claim 22 for the care of, or for protecting, the skin, more particularly for sun protection, **characterized in that** it is present as an emulsion, a dispersion, a suspension, an aqueous surfactant preparation, a milk, a lotion, a cream, a balm, an ointment, a gel, granules, a powder, a stick, a foam, an aerosol or a spray.

**24.** A cosmetic preparation as claimed in claim 22 or 23, **characterized in that** it contains other UV filters in the form of soluble compounds or other pigments.

**25.** A cosmetic preparation as claimed in any of claims 22 to 24, **characterized in that** it contains other skin-care components, other cosmetic active components, more particularly vitamins, skin moisturizers, antioxidants, antimicrobial agents, deodorizing or perspiration-inhibiting components and/or auxiliaries and additives.

**26.** The use of the cosmetic preparation claimed in any of claims 22 to 24 as a sun protection preparation.

**27.** The use of the cosmetic preparation claimed in any of claims 22 to 24, **characterized in that** the cosmetic preparation is topically applied.

**28.** The use of the surface-modified zinc oxide claimed in any of claims 1 to 8 or the zinc oxide dispersions claimed in claim 15 or 16 for UV protection.

**29.** The use of the surface-modified zinc oxide claimed in any of claims 1 to 8 or the zinc oxide dispersions claimed in claim 15 or 16 as an antimicrobial component.

**30.** A pharmaceutical preparation containing the surface-modified zinc oxide claimed in any of claims 1 to 8 or the zinc oxide dispersion claimed in claim 15 or 16.

## Revendications

1. Oxyde de zinc nanoparticulaire à surface modifiée, **caractérisé en ce que** la modification de surface comprend un revêtement par un acide d'oligo- ou de poly-(éthylèneglycol).

2. Oxyde de zinc à surface modifiée selon la revendication 1, **caractérisé en ce que** l'acide d'oligo- ou de poly-(éthylèneglycol) correspond à la formule générale $R-CH_2-(O-CH_2-CH_2)_n-O-CH_2-COOH$, n étant un nombre entier de 0 à 40.

3. Oxyde de zinc à surface modifiée selon la revendication 2, **caractérisé en ce que** R correspond à un résidu choisi parmi H, $CH_3$, $C_2H_5$, $C_3H_7$, $CH(CH_3)_2$, OH, $NH_2$, COOH, $CONH_2$, $CO_2CH_3$, $CO_2C_2H_5$, $CO_2C_3H_7$ et $CO_2CH(CH_3)_2$.

4. Oxyde de zinc à surface modifiée selon l'une quelconque des revendications précédentes, **caractérisé en ce que** la surface est modifiée par un acide oligo- ou poly-(éthylèneglycol)-diacétique de formule générale $HOOC-CH_2-(O-CH_2-CH_2)_n-O-CH_2-COOH$ et n correspond à un nombre entier de 0 à 40.

5. Oxyde de zinc à surface modifiée selon la revendication 2, **caractérisé en ce que** la surface des particules d'oxyde de zinc est modifiée par l'acide poly(éthylèneglycol)-diacétique 600.

6. Oxyde de zinc à surface modifiée selon la revendication 2, **caractérisé en ce que** la surface est modifiée par l'acide 2-[2-(2-méthoxyéthoxy)-éthoxy]acétique.

7. Oxyde de zinc à surface modifiée selon l'une quelconque des revendications précédentes, **caractérisé en ce que** les particules primaires d'oxyde de zinc possèdent un diamètre de 1 à 200 nm.

8. Oxyde de zinc à surface modifiée selon l'une quelconque des revendications précédentes, **caractérisé en ce que** les particules primaires d'oxyde de zinc présentent un diamètre de 2 à 50 nm, en particulier de 3 à 10 nm.

9. Procédé pour préparer de l'oxyde de zinc nanoparticulaire à surface modifiée, **caractérisé en ce que**,

   a) de l'oxyde de zinc nontraité est mis en suspension dans un solvant polaire,
   b) ensuite, il est mélangé et chauffé avec un acide d'oligo- ou de polyéthylèneglycol selon l'une quelconque des revendications 4 à 9, et
   c) le solvant polaire est éliminé.

10. Procédé selon la revendication 9, **caractérisé en ce que**, dans l'étape de procédé b) l'oxyde de zinc non traité, dispersé, est mélangé et chauffé avec un acide oligo- ou polyéthylèneglycol de formule générale $R-CH_2-(O-CH_2-CH_2)_n-O-CH_2-COOH$, en particulier avec l'acide 2-[2-(2-méthoxyéthoxy)-éthoxy]acétique.

11. Procédé selon la revendication 9 ou 10, **caractérisé en ce que** le solvant est éliminé par évaporation à la pression normale ou sous-atmosphérique, par congélation, lyophilisation, filtration, suivies par un séchage ou séchage à une température élevée à la pression normale, ou de préférence à une pression réduite.

12. Procédé pour préparer des dispersions d'oxyde de zinc, **caractérisé en ce que** l'oxyde de zinc à surface modifiée selon l'une quelconque des revendications 1 à 8, est introduit dans de l'eau, dans un solvant organique ou dans un mélange d'un solvant organique et d'eau, et est dispersé à l'aide d'un procédé approprié.

13. Procédé selon la revendication 12, **caractérisé en ce que**, le solvant organique possède un moment dipolaire supérieur à 0,35 $\mu$/D.

14. Procédé selon la revendication 12 ou 13, **caractérisé en ce que** le solvant organique pour un mélange est choisi parmi le méthanol, l'éthanol, le n-propanol, le i-propanol, l'acétone, l'éther diéthylique, le diméthylsulfoxyde, le tétrahydrofurane, le chlorure de méthylène, le trichlorométhane-éthanol, l'acétate d'éthyle, l'acétate d'isobutyle, et/ou le toluène ou un mélange de ceux-c.

15. Dispersion d'oxyde de zinc, préparée d'après un procédé selon l'une quelconque des revendications 12 à 14, **caractérisé en ce que** la dispersion présente une teneur en oxyde de zinc dispersé, de 0,001 à 50% en poids.

**16.** Dispersion d'oxyde de zinc selon la revendication 15, **caractérisée en ce que**, la dispersion présente une teneur en oxyde de zinc dispersé de 0,1 à 10% en poids, en particulier de 1 à 5% en poids.

**17.** Procédé pour le revêtement de surface, **caractérisé en ce que** l'on applique une dispersion d'oxyde de zinc qui a été préparée d'après un procédé selon l'une quelconque des revendications 12 à 16, sur la surface à revêtir, puis on élimine le solvant.

**18.** Procédé selon la revendication 17, **caractérisé en ce qu'**un agent dopant est ajouté à la dispersion d'oxyde de zinc avant l'application sur la surface à revêtir.

**19.** Procédé selon la revendication 17 ou 18, **caractérisé en ce que** la dispersion d'oxyde de zinc est appliquée sur le substrat à revêtir dans un dispositif de revêtement par centrifugation (spin-coater).

**20.** Procédé selon l'une quelconque des revendications 17 à 19, **caractérisé en ce que** la surface revêtue est ensuite chauffée à une température entre 100 et 1 000°C, de préférence à l'abri de l'oxygène, ou sous une atmosphère réductrice.

**21.** Procédé selon l'une quelconque des revendications 17 à 20, pour préparer des surfaces et des couches électriquement conductrices.

**22.** Agent cosmétique, contenant un oxyde de zinc à surface modifiée, selon l'une quelconque des revendications 1 à 8, ou une dispersion d'oxyde de zinc, selon l'une quelconque des revendications 15 ou 16.

**23.** Agent cosmétique selon la revendication 22, pour soigner ou pour protéger la peau, en particulier pour la protection solaire, **caractérisé en ce que** la composition existe sous forme d'une émulsion, d'une dispersion, d'une suspension, d'une préparation aqueuse tensio-active, d'un lait, d'une lotion, d'une crème, d'un baume, d'une pommade, d'un gel, d'un granulat, d'une poudre, d'une préparation en stick, d'une mousse, d'un aérosol, ou d'un spray.

**24.** Agent cosmétique selon la revendication 22 ou 23, **caractérisé en ce que** l'agent contient d'autres filtres protégeant de la lumière UV sous forme de composés solubles ou d'autres pigments.

**25.** Agent cosmétique selon l'une quelconque des revendications 22 à 24, **caractérisé en ce qu'**il contient des substances de soins, d'autres ingrédients actifs cosmétiques, en particulier des vitamines, des hydratants de la peau, des anti-oxydants, des ingrédients actifs anti-microbiens, des substances désodorisantes ou anti-transpirantes, et/ou des adjuvants ou des additifs.

**26.** Utilisation d'un agent cosmétique selon l'une quelconque des revendications 22 à 24 sous forme d'agent de protection solaire.

**27.** Utilisation d'un agent cosmétique selon l'une quelconque des revendications 22 à 24, **caractérisée en ce que** l'agent cosmétique est appliqué de façon topique.

**28.** Utilisation d'oxyde de zinc à surface modifiée selon l'une quelconque des revendications 1 à 8 ou de dispersions d'oxyde de zinc selon l'une quelconque des revendications 15 ou 16 pour une protection contre les UV.

**29.** Utilisation d'oxyde de zinc à surface modifiée selon l'une quelconque des revendications 1 à 8, ou de dispersions d'oxyde de zinc selon l'une quelconque des revendications 15 ou 16, sous forme d'ingrédient actif anti-microbien.

**30.** Agent pharmaceutique, contenant un oxyde de zinc à surface modifiée selon l'une quelconque des revendications 1 à 8, ou une dispersion d'oxyde de zinc selon l'une quelconque des revendications 15 ou 16.

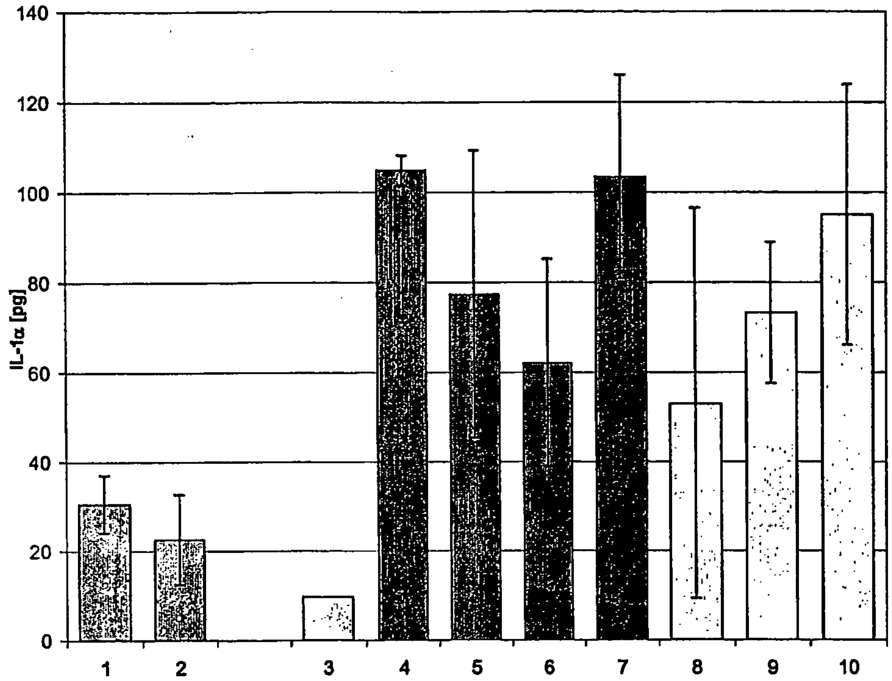


**FIG. 1:** Interleukin-1α-Ausschüttung der Epidermiskulturen nach 1-stündiger Vorschädigung mit SDS 0,16% und anschließender 20stündiger Inkubation mit den Prüfsubstanzen.

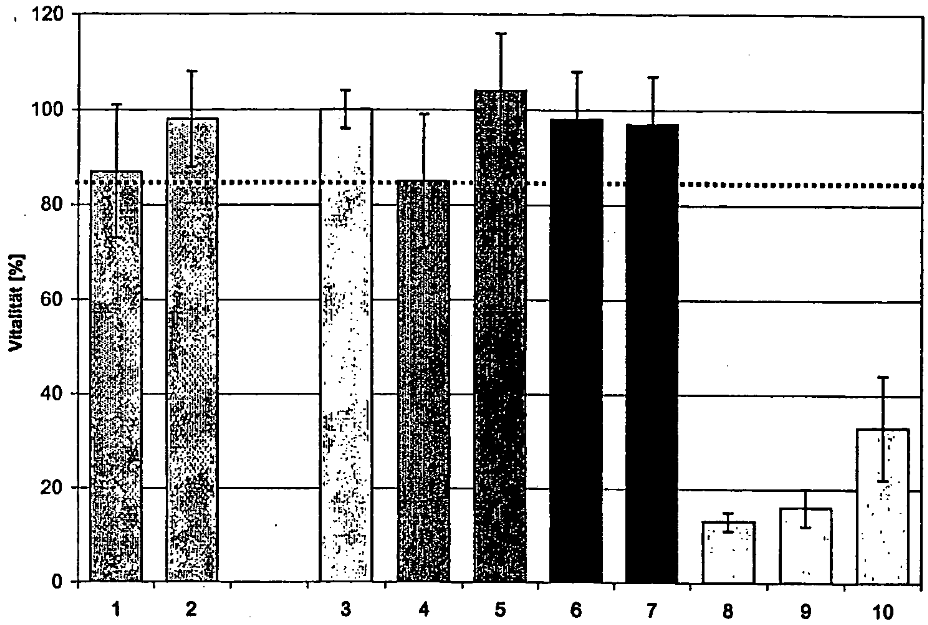


**FIG. 2:** Vitalität der Hautkulturen nach 1stündiger Vorschädigung mit SDS 0,16% und anschließender 20stündiger Inkubation mit den Prüfsubstanzen (bezogen auf die Negativkontrolle Aqua/Aqua=100%).